(19) Europäisches Patentamt
European Patent Office
Office européen des brevets

(11) Veröffentlichungsnummer: **0 051 565**
**A1**

(12) # EUROPÄISCHE PATENTANMELDUNG

(21) Anmeldenummer: 81810428.3

(22) Anmeldetag: 28.10.81

(51) Int. Cl.³: C 07 D 249/10
A 01 N 47/38

(30) Priorität: 03.11.80 CH 8155/80
25.09.81 CH 6203/81

(43) Veröffentlichungstag der Anmeldung:
12.05.82 Patentblatt 82/19

(84) Benannte Vertragsstaaten:
BE CH DE FR GB IT LI NL

(71) Anmelder: CIBA-GEIGY AG
Patentabteilung Postfach
CH-4002 Basel(CH)

(72) Erfinder: Kristiansen, Odd, Dr.
Delligrabenstrasse 7
CH-4313 Möhlin(CH)

(72) Erfinder: Drabek, Jozef
Dr. Benkenstrasse 12
CH-4104 Oberwil(CH)

(54) **1-N,N-Dimethylcarbamoyl-3(5)-cyclopropyl-1,2,4-triazole, Verfahren zu ihrer Herstellung und ihre Verwendung in der Schädlingsbekämpfung.**

(57) 1-N,N-Dimethylcarbamoyl-3(5)-cyclopropyl-1,2,4-triazole der Formel

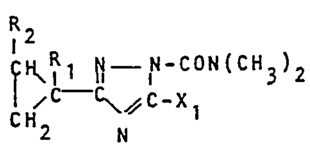

oder

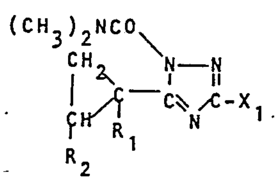

worin $R_1$ und $R_2$ je Wasserstoff oder $C_1$-$C_4$-Alkyl und $X_1$ Halogen bedeuten. Ein Verfahren zur Herstellung dieser Triazole und ihre Verwendung in der Schädlingsbekämpfung werden beschrieben.

EP 0 051 565 A1

CIBA-GEIGY AG 5-13133/1+2

Basel (Schweiz)

1-N,N-Dimethylcarbamoyl-3(5)-cyclopropyl-1,2,4-triazole, Verfahren zu ihrer Herstellung und ihre Verwendung in der Schädlingsbekämpfung

Die vorliegende Erfindung betrifft 1-N,N-Dimethylcarbamoyl-3(5)-cyclopropyl-1,2,4-triazole, ein Verfahren zu ihrer Herstellung und ihre Verwendung in der Schädlingsbekämpfung.

Die 1-N,N-Dimethylcarbamoyl-3(5)-cyclopropyl-1,2,4-triazole haben die Formeln

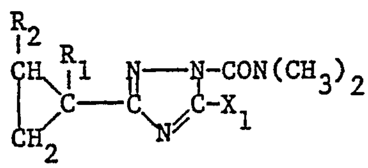 (I) oder 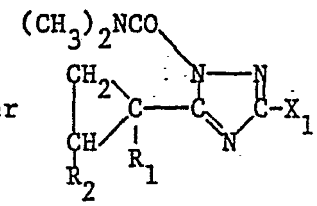 (Ia)

worin $R_1$ und $R_2$ je Wasserstoff oder $C_1$-$C_4$-Alkyl und $X_1$ Halogen bedeuten.

Unter Halogen ist dabei Fluor, Chlor, Brom oder Jod, insbesondere aber Chlor oder Brom zu verstehen. Die bei $R_1$ und $R_2$ in Frage kommenden Alkylgruppen sind: Methyl, Aethyl, Propyl, Isopropyl, n-, i-, sek.- und tert.-Butyl.

Wegen ihrer Wirkung bevorzugt sind Verbindungen der Formeln I oder Ia, worin $R_1$ und $R_2$ je Wasserstoff oder Methyl und $X_1$ Chlor oder Brom bedeuten.

Die Verbindungen der Formeln I oder Ia werden nach an sich bekannten Methoden hergestellt, indem man z.B. eine Verbindung der Formel

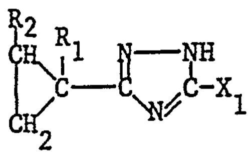 (II) oder 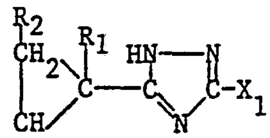 (IIa)

worin $R_1$, $R_2$ und $X_1$ die für die Formeln I und Ia angegebene Bedeutun haben, in Gegenwart einer Base mit einem N,N-Dimethylcarbamoylhalogenid, insbesondere N,N-Dimethylcarbamoylchlorid, reagieren lässt.

Das Verfahren wird bei einer Reaktionstemperatur zwischen -50°C und +130°C, vorzugsweise zwischen -10°C und +100°C, bei normalem oder leicht erhöhtem Druck und in Gegenwart eines gegenüber den Reaktions teilnehmern inerten Lösungs- oder Verdünnungsmittels vorgenommen.

Als geeignete Basen für das Verfahren kommen insbesondere tertiäre Amine, wie Trialkylamine, Pyridine und Dialkylaniline, ferner Hydroxide, Oxide, Carbonate und Bicarbonate von Alkali- und Erdalkalimetallen sowie Alkalimetallalkoholate wie z.B. Kalium-tert.-butylat und Natriummethylat in Betracht.

Als Lösungs- oder Verdünnungsmittel eignen sich z.B. Aether und äth artige Verbindungen wie Diäthyläther, Di-isopropyläther, Dioxan, Tetrahydrofuran; aliphatische und aromatische Kohlenwasserstoffe, insbesondere Benzol, Toluol, Xylole; Ketone wie Aceton, Methyläthyl keton und Cyclohexanon; und Nitrile wie Acetonitril.

Die Ausgangsstoffe der Formeln II und IIa können analog bekannten Methoden hergestellt werden.

Die Verbindungen der Formeln I und Ia eignen sich zur Bekämpfung v Schädlingen an Tieren und Pflanzen.

Insbesondere eignen sich die Verbindungen der Formeln I und Ia zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera und von Milben und Zecken der Ordnung Akarina.

Vor allem eignen sich Verbindungen der Formeln I und Ia zur Bekämpfung von pflanzenschädigenden Insekten, insbesondere pflanzenschädigenden Frassinsekten, in Zier- und Nutzpflanzen, z.B. in Baumwoll- und Reiskulturen (z.B. gegen Spodoptera littoralis, Heliothis virescens und Laodelphax striatellus) und Gemüsekulturen (z.B. gegen Leptinotarsa decemlineata und Myzus persicae).

In diesem Zusammenhang ist hervorzuheben, dass die genannten Verbindungen sich sowohl durch eine stark ausgeprägte systemische als auch Kontakt-Wirkung gegen saugende Insekten insbesondere gegen saugende Insekten der Ordnung Homoptera und vor allem gegen Insekten der Familie Aphididae (wie z.B. Aphis fabae, Aphis craccivora und Myzus persicae), welche sich mit bekannten Mitteln nur schwierig bekämpfen lassen, auszeichnen.

Wirkstoffe der Formeln I und Ia zeigen auch eine sehr günstige Wirkung gegen Fliegen, wie z.B. Musca domestica und Mückenlarven. Daneben zeichnen sich die Verbindungen der Formel I durch eine breite ovizide und ovilarvizide Wirkung aus.

Darüber hinaus besitzen die Verbindungen der Formeln I und Ia fungizide und pflanzenregulatorische Eigenschaften und eine wertvolle Wirkung gegen pflanzenparasitäre Nematoden sowie gegen ektoparasitäre Milben und Zecken z.B. der Familien Ixodidae, Argasidae und Dermanyssidae.

Die Verbindungen der Formeln I und Ia werden in unveränderter Form oder vorzugsweise zusammen mit den in der Fomulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formeln I und Ia sowie gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht

sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formeln I und Ia nichtionogene, kation- und/oder anionaktive Tenside mit guten Emulgier-, Dispergier- und Netzeigenschaften in Betracht. Unter Tensiden sind auch Tensidgemischte zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- und Stearinsäure, oder von natürlichen Fettsäuregemischen, die z.B. aus Kokosnuss- oder Talgöl gewonnen werden. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatomen im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltende Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hdroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

"Mc Cutcheon's Detergents and Emulsifiers Annual" MC Publishing Corp., Ringwood, New Jersey, 1979.

Die pestiziden Zubereitungen enthalten in der Regel 0,1 bis 99%, insbesondere 0,1 bis 95%, Wirkstoff der Formel I und Ia, 1 bis 99,9% eines festen oder flüssigen Zusatzstoffes und 0 bis 25%, insbesondere 0,1 bis 25%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

<u>Formulierungsbeispiele für flüssige Wirkstoffe der Formeln I und Ia (% = Gewichtsprozent)</u>

| 1. <u>Emulsions-Konzentrate</u> | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | - | - |
| Tributylphenol-polyäthylenglykoläther (36 Mol AeO) | - | 12% | 4,2% |

| | | | |
|---|---|---|---|
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

| 3. Granulate | a) | b) |
|---|---|---|
| Wirkstoff | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formeln I und Ia (% = Gewichtsprozent)

5. Spritzpulver

| | a) | b) |
|---|---|---|
| Wirkstoff | 20% | 60% |
| Na-Ligninsulfonat | 5% | 5% |
| Na-Laurylsulfat | 3% | - |
| Na-Diisobutylnaphthalinsulfonat | - | 6% |
| Octylphenolpolyäthylenglykoläther (7-8 Mol AeO) | - | 2% |
| Hochdisperse Kieselsäure | 5% | 27% |
| Kaolin | 67% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

7. Stäubemittel

| | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1: Herstellung von 1-N,N-Dimethylcarbamoyl-3(5)-cyclopropyl-5(3)-brom-1,2,4-triazol. Eine Suspension von 15 g 3-Cyclopropyl-5-brom-1,2,4-triazol, 11,1 g Kaliumkarbonat und 12,9 g Dimethylcarbamoylchlorid in 200 ml Aceton werden 5 Stunden unter Rückfluss gekocht. Nach dem Abkühlen, Abfiltrieren und Eindampfen wird das Rohprodukt in 200 ml Chloroform gelöst und mit 2 x 50 ml Wasser ausgeschüttelt. Nach dem Abdestillieren des Lösungsmittels und des überschüssigen Dimethylcarbamoylchlorids erhält man nach der chromatographischen Reinigung die Verbindung der Formel

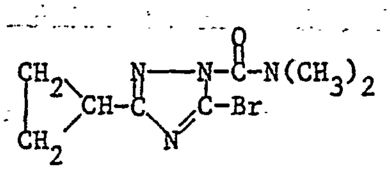

mit einem Brechungsindex von $n_D^{20} = 1.5445$.

Auf analoge Weise werden auch folgende Verbindungen hergestellt:

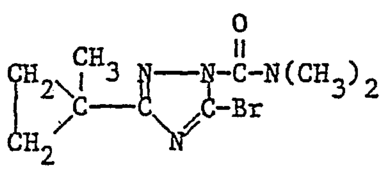

$n_D^{20°} = 1.5325$

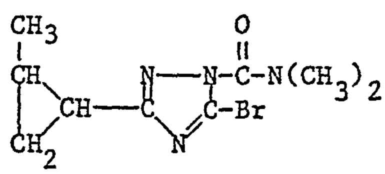

$n_D^{20°} = 1.5343$

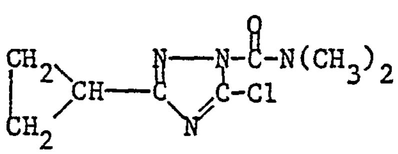

Smp.: 86-87°C.

Beispiel 2: Insektizide Kontakt-Wirkung: Myzus persicae

In Wasser angezogene Pflanzen (Vicia faba) werden vor dem Versuchsbeginn je mit ca. 200 Individuen der Spezies Myzus persicae besiedelt. Die so behandelten Pflanzen werden 3 Tage später mit einer Lösung enthaltend 10 oder 1 ppm der zu prüfenden Verbindung aus 30 cm Distanz bis zur Tropfnässe besprüht. Man verwendet pro Test-Verbindung und pro Konzentration zwei Pflanzen, und eine Auswertung der erzielten Abtötungsrate erfolgt nach weiteren 24 Stunden.

Innerhalb der oben angegebenen Konzentrationsgrenzen zeigen die Verbindungen gemäss Beispiel 1 die in der folgenden Tabelle angegebene Wirkung gegen Insekten der Spezies Myzus persicae.

Beispiel 3: Insektizide systemische Wirkung: Aphis craccivora

Bewurzelte Bohnenpflanzen werden in Töpfe, welche 600 ccm Erde enthalten verpflanzt. Anschliessend werden 50 ml einer Versuchslösung, 25 ppm, 5 ppm bzw. 1 ppm der zu prüfenden Verbindung direkt auf die Erde gegossen.

Nach 24 Stunden werden auf die oberirdischen Pflanzenteile Blattläuse (Aphis craccivora) gesetzt und die Pflanzen mit einem unten zugeschnürten Plastikzylinder überstülpt, um die Läuse vor einer eventuellen Kontakt- oder Gaswirkung der Testsubstanz zu schützen.

Die Auswertung der erzielten Abtötung erfolgt 24 und 48 Stunden nach Versuchsbeginn. Pro Konzentrationsdosis Testsubstanz werden zwei Pflanzen, je eine in einem separaten Topf, verwendet. Der Versuch wird bei 25°C und 70% relativer Luftfeuchtigkeit durchgeführt.

Innerhalb der oben angegebenen Konzentrationsgrenzen zeigen Verbindungen gemäss Beispiel 1 die in der folgenden Tabelle angegebene Wirkung gegen Insekten der Spezies Aphis craccivora.

Biologische Versuchsergebnisse

In der folgenden Tabelle sind Versuchsergebnisse auf der Basis der vorstehenden Beispiele aufgeführt, und zwar mit folgendem Bewertungsindex in Bezug auf die prozentuale Abtötung der Schädlinge:

A: 70-100% Abtötung bei 1 ppm Wirkstoffkonzentration
B: 70-100% Abtötung bei 5 ppm Wirkstoffkonzentration
C: 70-100% Abtötung bei 10 ppm Wirkstoffkonzentration
D: 70-100% Abtötung bei 25 ppm Wirkstoffkonzentration.

| Verbindungen | Wirksamkeit Mycus persicae | Aphis craccivora |
|---|---|---|
| 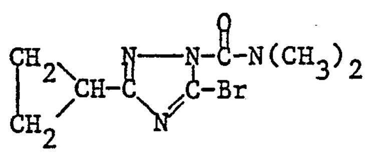 | A | A |
| 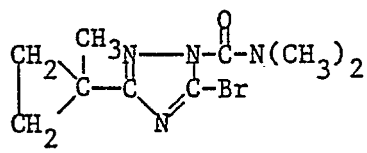 | A | A |
| 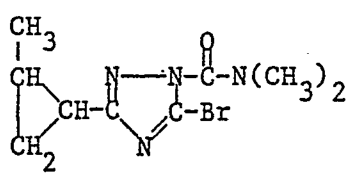 | C | B |
| 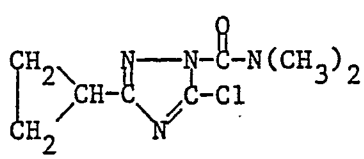 | A | A |

Patentansprüche

1. Ein 1-N,N-Dimethylcarbamoyl-3(5)-cyclopropyl-1,2,4-triazol der Formel

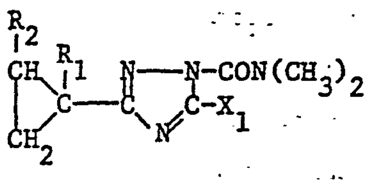

oder

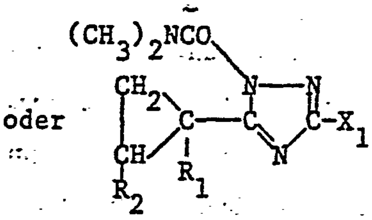

worin $R_1$ und $R_2$ je Wasserstoff oder $C_1$-$C_4$-Alkyl und $X_1$ Halogen bedeuten.

2. Eine Verbindung gemäss Anspruch 1, worin $R_1$ und $R_2$ je Wasserstoff oder Methyl und $X_1$ Chlor oder Brom bedeuten.

3. Die Verbindung gemäss Anspruch 2 der Formel

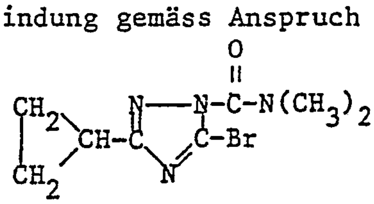

.

4. Die Verbindung gemäss Anspruch 2 der Formel

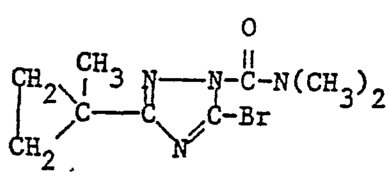

.

5. Die Verbindung gemäss Anspruch 2 der Formel

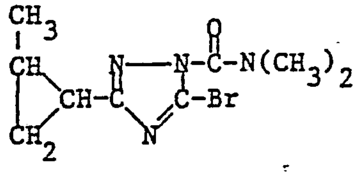

.

6. Die Verbindung gemäss Anspruch 2 der Formel

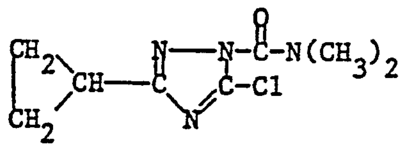

.

7. Verfahren zur Herstellung von Verbindungen gemäss Anspruch 1, dadurch gekennzeichnet, dass man in Gegenwart einer Base eine Verbindung der Formeln

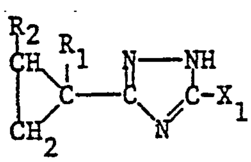

oder

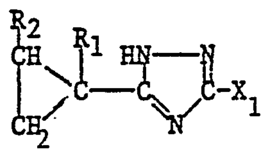

worin $R_1$, $R_2$ und $X_1$ die im Anspruch 1 angegebene Bedeutung haben, mit einem N,N-Dimethylcarbamoylhalogenid, insbesondere mit N,N-Dimethylcarbamoylchlorid, reagieren lässt.

8. Ein Schädlingsbekämpfungsmittel, welches als aktive Komponente eine Verbindung gemäss Anspruch 1 enthält.

9. Die Verwendung einer Verbindung gemäss Anspruch 1 zur Bekämpfung von Schädlingen an Tieren und Pflanzen.

10. Die Verwendung gemäss Anspruch 9 zur Bekämpfung von Insekten und Vertretern der Ordnung Akarina.

0051565

Europäisches Patentamt

# EUROPÄISCHER RECHERCHENBERICHT

Nummer der Anmeldung

EP 81 81 0428

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | GB - A - 2 011 414 (THE BOOTS CO.)<br>* Seiten 1-3 *<br>--- | 1,7-9 |
| | US - A - 3 308 131 (B.C. McKUSICK)<br>* Spalten 1-4 *<br>-------- | 1,7-9 |

KLASSIFIKATION DER ANMELDUNG (Int. Cl.³)

C 07 D 249/10
A 01 N 47/38

RECHERCHIERTE SACHGEBIETE (Int. Cl.³)

C 07 D 249/10
A 01 N 47/38

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 25.01.1982 | BRIGHENTI |

EPA form 1503.1 06.78